(19) **Europäisches Patentamt / European Patent Office / Office européen des brevets**

(11) **EP 4 556 553 A1**

(12) **EUROPEAN PATENT APPLICATION**
published in accordance with Art. 153(4) EPC

(43) Date of publication:
**21.05.2025 Bulletin 2025/21**

(21) Application number: **23855130.3**

(22) Date of filing: **14.08.2023**

(51) International Patent Classification (IPC):
**C12N 1/12** (2006.01)         **C12P 23/00** (2006.01)
**C12P 7/6434** (2022.01)        **C12P 7/6432** (2022.01)
**C12P 21/00** (2006.01)         **A23K 10/16** (2016.01)
**A23L 29/00** (2016.01)         **C12R 1/89** (2006.01)

(52) Cooperative Patent Classification (CPC):
**A23K 10/16; A23L 29/00; C12N 1/12;**
**C12P 7/6432; C12P 7/6434; C12P 21/00;**
**C12P 23/00;** C12R 2001/89

(86) International application number:
**PCT/KR2023/012051**

(87) International publication number:
**WO 2024/039163 (22.02.2024 Gazette 2024/08)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **18.08.2022 KR 20220103398**

(71) Applicant: **CJ CheilJedang Corporation**
**Seoul 04560 (KR)**

(72) Inventors:
• **JANG, Sunghoon**
  **Seoul 04560 (KR)**
• **SHIN, Won Sub**
  **Seoul 04560 (KR)**
• **CHOI, Jung-Woon**
  **Seoul 04560 (KR)**

• **KANG, Hae-Won**
  **Seoul 04560 (KR)**
• **RYU, Ae Jin**
  **Seoul 04560 (KR)**
• **GWAK, Jun Seok**
  **Seoul 04560 (KR)**
• **KIM, Ji Young**
  **Seoul 04560 (KR)**

(74) Representative: **Meissner Bolte Partnerschaft**
**mbB**
**Patentanwälte Rechtsanwälte**
**Postfach 86 06 24**
**81633 München (DE)**

Remarks:
The complete document including Reference Table(s) and the Sequence Listing(s) can be downloaded from the EPO website

(54) **NOVEL SCHIZOCHYTRIUM SP. STRAIN FOR PRODUCING BIOMASS CONTAINING HIGH PROTEIN CONTENT, ANTIOXIDANT PIGMENTS AND OMEGA-3 FATTY ACIDS, AND USE THEREOF**

(57)     The present invention relates to a novel *Schizochytrium* sp. strain containing a high protein content, antioxidant pigments and omega-3 fatty acids, and a method for producing a biomass containing a high protein content, antioxidant pigments and omega-3 fatty acids by using the strain. The novel *Schizochytrium* sp. strain of the present invention simultaneously contains, in the cells thereof, a high content of protein ingredients including amino acids such as glutamic acid, arginine and aspartic acid, antioxidant pigments of beta-carotene and canthaxanthin, and omega-3 unsaturated fatty acid ingredients including eicosapentaenoic acid and docosahexaenoic acid, and thus the strain itself or the biomass and bio-oil produced by culturing and fermenting the strain can be effectively used as a feed composition or a food composition and the like.

**(Cont. next page)**

EP 4 556 553 A1

【FIG. 1】

**Description**

[TECHNICAL FIELD]

Cross-reference to related application(s)

The present application claims the benefit of the priority based on Korean Patent Application No. 10-2022-0103398 filed on August 18, 2022, and the entire contents disclosed in the documents of the corresponding Korean patent application are incorporated as a part of the present description.

The present invention relates to a novel *Schizochytrium* sp. strain producing a biomass comprising high protein content, antioxidant pigments and omega-3 fatty acid, and a method for producing a biomass containing a high protein content, antioxidant pigments and omega-3 fatty acid using thereof.

[BACKGROUND ART]

In order for organisms to maintain life and grow and develop, they should ingest and use various kinds of nutritional substances in the body. Nutritional substances can be largely classified into calorific substances such as sugars and lipid components, which are mainly used for calories, structural nutrients such as proteins and minerals, and regulatory nutrients such as vitamins and the like. Among them, the protein component performs various kinds of important roles such as components in living organisms, a catalytic role as enzymes of various kinds of chemical reactions in cells, immunity by forming antibodies, and the like.

Animal protein sources with nutritionally excellent quality have been consumed while forming the biggest market, but recently, vegetable protein sources have been getting a lot of attention, along with responses to environmental issues such as population growth and greenhouse gas reduction and the like and global social changes such as animal welfare enhancement and the like. The best-known vegetable protein source is soybean, and it is utilized as a food protein in itself, or soybean meal which is a residue left after extracting oil for food is utilized as animal feed. However, as the demand of soybean protein increases, it is not free from issues such as lack of biodiversity according to soybean intensive cultivation and damage occurrence to other plants according thereto, and expansion of genetically modified (GMO) seeds, and the like. In addition, when used as feed, there are problems such as low digestibility due to the high carbohydrate composition ratio, and low protein absorption and low mineral availability due to the high phytic acid content, and the like. Furthermore, there are still contamination issues of pesticide residue components used during soybean cultivation and harmful microorganisms such as other fungi and the like.

As an alternative to this, a protein in a form of SCP (Single Cell Protein) using microalgae, which is a phytoplankton is attracting attention. In particular, spirulina and chlorella have been well known. It is nutritionally excellent as it contains protein components of various amino acid compositions including essential amino acids, but there is a disadvantage in that the price of supply increases due to the non-palatability for unique odor (臭) and taste derived from the chlorophyll component and the culturing and producing method through photosynthesis.

In the present research, in order to suggest a new material capable of supplementing disadvantages of such a conventional vegetable protein source, a vegetable protein source derived from a *Schizochytrium* sp. strain which is the marine microalgae has been tried to be developed. The *Schizochytrium* sp. strain is the marine microalgae, and has no chlorophyll in the body, and can be cultured in a fermentor by a conventional dependent fermentation method. Through this, it is free from pesticide residues and other pollution source issues, and mass production with consistent quality products is possible. In addition, there is an advantage in that unsaturated fatty acids of omega-3 components including DHA and EPA can be contained in cells, and therefore, there is an advantage in that essential fatty acids can be further supplied differently from conventional soybean meal or other microorganism-derived SCPs. In addition, it has an advantage of high digestibility in the body due to a characteristic of having a cell membrane having proteins as a main component without cell walls.

Based on such advantages, recently, research and industrialization on production of polyunsaturated fatty acids including omega-3 unsaturated fatty acids such as docosahexaenoic acid (DHA), eicosapentaenoic acid (EPA), arachidonic acid (ARA), docosapentaenoic acid (DPA), and α-linolenic acid, and the like using microalgae have been proceeded very rapidly. As one example, U.S. Patent No. 5,130,242 discloses a method for preparing omega-3 polyunsaturated fatty acids using *Schizochytrium* sp. microorganisms, *Schizochytrium* sp. ATCC20888 and *Schizochytrium* sp. PTA10208.

The present invention provides a novel *Schizochytrium* sp. mutant strain which comprises antioxidant pigments in addition to high content proteins and omega-3 fatty acids simultaneously and/or producing them, and suggests the possibility of mass production as a new vegetable protein material through this.

[DISCLOSURE]

[TECHNICAL PROBLEM]

One embodiment of the present application provides a novel *Schizochytrium* sp. CD03-7004 strain (Accession number KCTC15006BP).

Another embodiment of the present application provides a biomass comprising the *Schizochytrium* sp. strain, a culture solution of the strain, a dry matter of the culture solution, or a lysate of the dry matter.

Other embodiment of the present application provides a feed composition comprising the *Schizochytrium* sp. strain, a culture solution of the strain, a dry matter of the culture solution, or a lysate of the dry matter.

Other embodiment of the present application provides a food composition comprising the *Schizochytrium* sp. strain, a culture solution of the strain, a dry matter of the culture solution, or a lysate of the dry matter.

Other embodiment of the present application provides an antioxidant composition comprising the *Schizochytrium* sp. strain, a culture solution of the strain, a dry matter of the culture solution, or a lysate of the dry matter. Other embodiment of the present application provides a method for producing antioxidant pigments, comprising

culturing the *Schizochytrium* sp. strain; and
extracting carotenoid-based antioxidant pigments from a culture of the strain.

[TECHNICAL SOLUTION]

Each description and embodiment disclosed in the present application can be applied to each of other description and embodiments. In other words, all combinations of various elements disclosed in the present application fall within the scope of the present invention. In addition, it cannot be seen that the scope of the present invention is limited by the detailed description described below. Furthermore, those skilled in the art will recognize or confirm many equivalents to specific aspects of the present invention described in the present application using only a common experiment. In addition, such equivalents are intended to be included in the present invention.

One embodiment of the present application provides microalgae, novel *Schizochytrium* sp. CD03-7004 strain (Accession number KCTC15006BP).

The term used in the present description, "Thraustochytrid" means an order *Thraustochytriales* microalgae. In addition, the term used in the present description, *"Schizochytrium* sp." is one of the genus names belonging to the family *Thraustochytriaceae* of the order *Thraustochytriales,* and may be interchangeably used with the term *"Schizochytrium* sp. (genus *Schizochytrium*)"*. Furthermore, the term "microalgae" may be interchangeably used with "strain" in the present description, and is a small algae that cannot be seen with naked eyes and can be seen through a microscope, and refers to an organism which lives freely floating in water. There are various types on the microalgae, and it includes even strains that cannot photosynthesize and grow only as heterotroph.

As one embodiment in the present application, gamma rays were irradiated to the wild-type *Schizochytrium* sp. CD01-5000 strain to cause mutation, and a strain with producing ability of antioxidant pigments in the mutant strains was selected, and this was named *Schizochytrium* sp. CD03-7004, and deposited to Korean Collection for Type Cultures (KCTC) which is an international depository authority under the Budapest Treaty on June 20, 2022 and was given an accession number KCTC15006BP.

The *Schizochytrium* sp. CD03-7004 strain produces and/or comprises carotenoid-based antioxidant pigments with high efficacy of antioxidation, anti-inflammation, and the like, or has producing ability of carotenoid-based antioxidant pigments.

The carotenoid-based antioxidant pigments may be beta-carotene or canthaxanthin, but not limited thereto.

"Producing ability" used in the present description includes increase in total protein amount per unit population of microalgae or unit dry weight, increase in amount of polyunsaturated fatty acids, increase of omega-3 unsaturated fatty acids, or increase in amount of docosahexaenoic acid (DHA) and eicosapentaenoic acid (EPA) or increase in relative ratio of polyunsaturated fatty acids among produced fatty acids, increase in relative ratio of omega-3 unsaturated fatty acids among produced total fatty acids or polyunsaturated fatty acids, or increase in relative ratio of amount of docosahexaenoic acid and eicosapentaenoic acid; or increase in total biomass amount per unit population or unit dry matter of microalgae, or increase in amount of total carotenoid-based antioxidant pigments per unit population or unit dry matter of microalgae, increase in amount of beta-carotene and canthaxanthin, or increase in relative ratio of beta-carotene and canthaxanthin among produced carotenoid-based antioxidant pigments. Any one of the above may lead to increase in production of the desired useful substance, and for example, when the relative ratio increases, not only the desired useful substance increases, but also the concentration increases, so separation may be facilitated.

The term used in the present description, "carotenoid" is C40 isoprenoid compounds with antioxidant activity, and is present in various colors such as yellow, red, orange, and the like depending on the molecular structure. The carotenoid is known to have an antioxidant effect capable of preventing damages due to oxidation, an effect or preventing or treating diseases including cancer, and an effect of reducing skin damages from exposure to ultraviolet rays or inhibiting melanin production, so it has been used in health food, food coloring agents, animal feed, and the like.

"Beta-carotene" of the present description is one of carotenoid-based antioxidant pigments, and is a precursor of almost all carotenoids. Beta-carotene is a precursor of vitamin A with yellow color, and is converted into vitamin A in the body, and has little effect on coloring of egg yolk or skin. Beta-carotene has been widely used as a food additive due to its antioxidant effect capable of preventing damages by oxidation, a high nutritional value and a unique color, and also, it can prevent adult diseases such as cancer, arteriosclerosis, arthritis, cataract, and the like caused by harmful oxygen, so it can be usefully used as an anti-cancer agent, a vascular disease agent, an eye disease agent, and an antioxidant related to skin aging, and the like.

"Canthaxanthin" of the present description is one kind of carotenoid pigments produced by oxidation of beta-carotene, and has an orange color, and is also called 4,4'-diketo-beta-carotene. Canthaxanthin has been sued as a cosmetic and a food coloring agent; and an antioxidant (Palozza, P et al., Arch. Biochem. Biophys., 297, 291-295, 1992; Palozza, P et al., Arch. Biochem. Biophys., 325, 145-151, 1996); an anticancer agent (Tanaka, T. et al., Cancer Res., 55, 4059-4064, 1995; Tanaka, T. et al., Carcinogenesis, 16, 2957-2963, 1995; Grubbs, C. J. et al., 48, 239-245, 1991; Katsumura, N. et al., Nutr. Cancer, 26, 203-208, 1996), and the like.

The *Schizochytrium* sp. strain produces and/or comprises high content omega-3 unsaturated fatty acids.

The omega-3 unsaturated fatty acid may be docosahexaenoic acid (DHA) or eicosapentaenoic acid (EPA), but not limited thereto.

The term used in the present description, "docosahexaenoic acid (DHA)" is one of polyunsaturated fatty acids having the chemical formula of $C_{22}H_{32}O_2$, and corresponds to omega-3 fatty acid together with alpha-linolenic acid ($\alpha$-linolenic acid: ALA) and eicosapentaenoic acid (EPA), and the common name is cervonic acid, and it may also be expressed as 22:6 n-3 as an abbreviation.

The term used in the present description, "eicosapentaenoic acid (EPA)" is one of polyunsaturated fatty acids having the chemical formula of $C_{20}H_{30}O_2$, and corresponds to omega-3 fatty acid together with ALA and DHA, and it may also be expressed as 20:5 n-3 as an abbreviation.

In one embodiment of the present application, the *Schizochytrium* sp. strain may produce and/or comprise docosahexaenoic acid of 35 to 60 % by weight based on the total weight of fatty acids. Specifically, the *Schizochytrium* sp. strain may produce and/or comprise docosahexaenoic acid of 35 to 60 % by weight, 40 to 60 % by weight, 42 to 60 % by weight, 45 to 60 % by weight, 49 to 60 % by weight, 35 to 55 % by weight, 40 to 55 % by weight, 42 to 55 % by weight, 45 to 55 % by weight, 35 to 50 % by weight, 40 to 50 % by weight, 42 to 50 % by weight, 45 to 50 % by weight, or 48 to 52 % by weight, based on the total weight of fatty acids.

In one embodiment of the present application, the *Schizochytrium* sp. strain may produce and/or comprise eicosapentaenoic acid of 0.1 to 5 % by weight based on the total weight of fatty acids. Specifically, the *Schizochytrium* sp. strain may produce and/or comprise eicosapentaenoic acid of 0.1 to 5 % by weight, 0.5 to 5 % by weight, 0.7 to 5 % by weight, 1 to 5 % by weight, 1.5 to 5 % by weight, 2 to 5 % by weight, 0.1 to 3 % by weight, 0.5 to 3 % by weight, 1 to 3 % by weight, 1.5 to 3 % by weight, 2 to 3 % by weight, 0.1 to 2.5 % by weight, 0.5 to 2.5 % by weight, 1 to 2.5 % by weight, 1.5 to 2.5 % by weight, 1.7 to 2.5 % by weight, 2 to 2.5 % by weight, or 1.8 to 2.4 % by weight, based on the total weight of fatty acids.

In addition, the present invention provides a biomass or bio-oil comprising the *Schizochytrium* sp. strain, a culture of the strain, a dry matter of the culture, or a lysate of the dry matter.

The description of the *Schizochytrium* sp. strain is as described above.

The term used in the present description, "biomass" means an energy source of bioenergy, that is, a living organism such as a plant, an animal, a microorganism, and the like usable as chemical energy, and also means the weight or energy amount of a specific living organism present in a unit time and space ecologically. In addition, the biomass includes compounds secreted by cells, but not limited thereto, and may contain not only extracellular substances but also cells and/or intracellular contents. In the present application, the biomass may be the *Schizochytrium* sp. strain itself, a culture thereof, a dry matter thereof, a lysate thereof, or a product produced by culturing or fermenting the strain, or may be a concentrate or dry matter of the biomass, but not limited thereto.

The "culture" of the *Schizochytrium* sp. microalga refers to a product produced by culturing the strain, and specifically, it may be a culture solution comprising the microalga or a culture filtrate from which the strain is removed from the culture solution, but not limited thereto. The "dry matter" of the *Schizochytrium* sp. strain culture is that moisture is removed in the culture of the strain, and it may be in a dried microbial cell of the strain, but not limited thereto. Moreover, the "lysate" of the dry matter collectively calls the result of lysing the dry matter from which moisture is removed from the culture of the strain, and for example, it may be a dried microbial cell powder, but not limited thereto. The culture of the *Schizochytrium* sp. strain may be prepared according to a culturing method known in the art by inoculating the strain to a microalgal culture medium, and the dry matter of the culture and lysate thereof may be also prepared according to a method for processing or drying of a strain or culture solution known in the art.

The term used in the present description, "bio-oil" means oil obtained from a biomass by biological, thermochemical and physicochemical extraction processes, and the bio-oil prepared in the present application may contain polyunsaturated fatty acids, and specifically, it may contain DHA and EPA, but not limited thereto.

In the present description, the bio-oil may comprise an extract of a biomass.

As a method for preparing the bio-oil extract, a method of utilizing an enzyme such as protease, cellulase, pectinase, or chitinase according to a method of crushing or lysing a cell membrane or cell wall component, a method of crushing a cell membrane or cell wall component physically using a homogenizer, an ultrasonicator, bead treatment, and the like, a method for extracting by directly adding a solvent and permeating into cells, a solvent-free extraction process of isolating through a centrifugation process after various crushing processes, and the like may be used, but not limited thereto.

The biomass derived from the *Schizochytrium* sp. strain may comprise carotenoid-based antioxidant pigments.

In one embodiment of the present application, the biomass derived from the *Schizochytrium* sp. strain may comprise carotenoid-based antioxidant pigments of 10 mg/kg or more, 20 mg/kg or more, 30 mg/kg or more, 40 mg/kg or more, 50 mg/kg or more, 60 mg/kg or more, 70 mg/kg or more, 80 mg/kg or more, 90 mg/kg or more, 100 mg/kg or more, 110 mg/kg or more, 10 to 200 mg/kg, 30 to 200 mg/kg, 50 to 200 mg/kg, 70 to 200 mg/kg, 90 to 200 mg/kg, 100 to 200 mg/kg, 110 to 200 mg/kg, 10 to 150 mg/kg, 30 to 150 mg/kg, 50 to 150 mg/kg, 70 to 150 mg/kg, 90 to 150 mg/kg, 100 to 150 mg/kg, 110 to 150 mg/kg, 10 to 120 mg/kg, 30 to 120 mg/kg, 50 to 120 mg/kg, 70 to 120 mg/kg, 90 to 120 mg/kg, 100 to 120 mg/kg, or 110 to 120 mg/kg, based on the total weight of biomass.

The carotenoid-based antioxidant pigments may be beta-carotene or canthaxanthin, but not limited thereto.

In one embodiment of the present application, the biomass derived from the *Schizochytrium* sp. strain may comprise beta-carotene of 0.1 mg/kg or more, 0.5 mg/kg or more, 1 mg/kg or more, 1.5 mg/kg or more, 2 mg/kg or more, 2.5 mg/kg or more, 0.1 to 5 mg/kg, 0.5 to 5 mg/kg, 1 to 5 mg/kg, 1.5 to 5 mg/kg, 2 to 5 mg/kg, 2.5 to 5 mg/kg, 0.1 to 4 mg/kg, 0.5 to 4 mg/kg, 1 to 4 mg/kg, 1.5 to 4 mg/kg, 2 to 4 mg/kg, 2.5 to 4 mg/kg, 0.1 to 3 mg/kg, 0.5 to 3 mg/kg, 1 to 3 mg/kg, 1.5 to 3 mg/kg, 2 to 3 mg/kg, or 2.5 to 3 mg/kg based on the total weight of biomass.

In one embodiment of the present application, the biomass derived from the *Schizochytrium* sp. strain may comprise canthaxanthin of 10 mg/kg or more, 20 mg/kg or more, 30 mg/kg or more, 40 mg/kg or more, 50 mg/kg or more, 60 mg/kg or more, 70 mg/kg or more, 80 mg/kg or more, 90 mg/kg or more, 100 mg/kg or more, 110 mg/kg or more, 10 to 200 mg/kg, 30 to 200 mg/kg, 50 to 200 mg/kg, 70 to 200 mg/kg, 90 to 200 mg/kg, 100 to 200 mg/kg, 110 to 200 mg/kg, 10 to 150 mg/kg, 30 to 150 mg/kg, 50 to 150 mg/kg, 70 to 150 mg/kg, 90 to 150 mg/kg, 100 to 150 mg/kg, 110 to 150 mg/kg, 10 to 120 mg/kg, 30 to 120 mg/kg, 50 to 120 mg/kg, 70 to 120 mg/kg, 90 to 120 mg/kg, 100 to 120 mg/kg, or 110 to 120 mg/kg based on the total weight of biomass.

The biomass derived from the *Schizochytrium* sp. strain may comprise high content proteins.

In one embodiment of the present application, the biomass derived from the *Schizochytrium* sp. strain may comprise a crude protein of 60 % by weight or more, 70 % by weight or more, 75 % by weight or more, 60 to 90 % by weight, 65 to 90 % by weight, 70 to 90 % by weight, 75 to 90 % by weight, 60 to 85 % by weight, 65 to 85 % by weight, 70 to 85 % by weight, 75 to 85 % by weight, 60 to 80 % by weight, 65 to 80 % by weight, 70 to 80 % by weight, or 75 to 80 % by weight based on the total weight of biomass.

The biomass derived from the *Schizochytrium* sp. strain may comprise high content omega-3 fatty acids.

In one embodiment of the present application, the biomass derived from the *Schizochytrium* sp. strain may comprise omega-3 unsaturated fatty acids of 42 % by weight or more, 45 % by weight or more, 50 % by weight or more, 52 % by weight or more, 55 % by weight or more, 60 % by weight or more, 65 % by weight or more, 70 % by weight or more, 42 to 70 % by weight, 45 to 70 % by weight, 50 to 70 % by weight, 52 to 70 % by weight, 55 to 70 % by weight, 42 to 65 % by weight, 45 to 65 % by weight, 50 to 65 % by weight, 52 to 65 % by weight, 55 to 65 % by weight, 42 to 60 % by weight, 45 to 60 % by weight, 50 to 60 % by weight, 52 to 60 % by weight, or 55 to 60 % by weight based on the total weight of fatty acids.

The omega-3 unsaturated fatty acids may be docosahexaenoic acid (DHA) or eicosapentaenoic acid (EPA), but not limited thereto.

In one embodiment of the present application, the biomass derived from the *Schizochytrium* sp. strain may comprise docosahexaenoic acid of 5 to 60 % by weight, 40 to 60 % by weight, 42 to 60 % by weight, 45 to 60 % by weight, 49 to 60 % by weight, 35 to 55 % by weight, 40 to 55 % by weight, 42 to 55 % by weight, 45 to 55 % by weight, 35 to 50 % by weight, 40 to 50 % by weight, 42 to 50 % by weight, 45 to 50 % by weight, or 48 to 52 % by weight based on the total weight of fatty acids.

In one embodiment of the present application, the biomass derived from the *Schizochytrium* sp. strain may comprise eicosapentaenoic acid of 0.1 to 5 % by weight, 0.5 to 5 % by weight, 0.7 to 5 % by weight, 1 to 5 % by weight, 1.5 to 5 % by weight, 2 to 5 % by weight, 0.1 to 3 % by weight, 0.5 to 3 % by weight, 1 to 3 % by weight, 1.5 to 3 % by weight, 2 to 3 % by weight, 0.1 to 2.5 % by weight, 0.5 to 2.5 % by weight, 1 to 2.5 % by weight, 1.5 to 2.5 % by weight, 1.7 to 2.5 % by weight, 2 to 2.5 % by weight, or 1.8 to 2.4 % by weight based on the total weight of fatty acids.

The genomic DNA of the *Schizochytrium* sp. CD03-7004 strain may comprise a nucleotide sequence comprising the nucleotide sequence of SEQ ID NO: 5 (TGCTTGCTTGCTTTT) contiguously twice.

The genomic DNA of the *Schizochytrium* sp. CD03-7004 strain may comprise a nucleotide sequence comprising the nucleotide sequence of SEQ ID NO: 1, and more specifically, may comprise the nucleotide sequence of SEQ ID NO: 1.

In the present description, the nucleotide sequence comprising the sequence of SEQ ID NO: 1(more specifically, the nucleotide sequence of SEQ ID NO: 1) can be identified using a primer set, a probe, or the like, and specifically, can be identified using a primer set, and more specifically, can be identified using a primer set comprising a primer having the sequence of SEQ ID NO: 3 and a primer having the sequence of SEQ ID NO: 4.

The *Schizochytrium* sp. CD03-7004 strain can be identified (screened, selected) from the wild-type strain using a primer set comprising a primer having the sequence of SEQ ID NO: 3 and a primer having the sequence of SEQ ID NO: 4.

In one embodiment of the present application, by comparing the complete genome sequences of the *Schizochytrium* sp. CD03-7004 strain and the wild-type *Schizochytrium* sp. CD01-5000 strain, it was confirmed that the *Schizochytrium* sp. CD03-7004 strain has a 15 base pairs (bp) additional region in the genome of the wild-type CD01-5000 strain. As a result of PCR using a primer set capable of amplifying a DNA fragment comprising the 15 bp nucleotide sequence region, it was confirmed that the *Schizochytrium* sp. CD03-7004 mutant strain can be distinguished (identified, screened, selected) from the wild-type *Schizochytrium* sp. CD01-5000 strain (the parent strain of the *Schizochytrium* sp. CD03-7004 mutant strain).

The present application provides a primer set for identifying *Schizochytrium* sp. CD03-7004 strain.

The primer set may be a primer set comprising a primer having the nucleotide sequence of SEQ ID NO: 3 and a primer having the nucleotide sequence of SEQ ID NO: 4.

The sequence amplified by the primer set may be the nucleotide sequence represented by SEQ ID NO: 1 and/or SEQ ID NO: 2.

By comparing the size of the DNA fragments amplified using the primer set, the *Schizochytrium* sp. CD03-7004 mutant strain can be distinguished (identified, screened, selected) from the wild-type *Schizochytrium* sp. CD01-5000 strain.

Another aspect of the present application provides a composition comprising the *Schizochytrium* sp. CD03-7004 strain, a culture of the strain, a dry matter of the culture, or a lysate of the dry matter.

The composition may comprise the *Schizochytrium* sp. strain, a biomass derived from the strain, bio-oil derived from the strain, or a combination thereof.

The composition may be in a form of solution, powder, or suspension, but not limited thereto. The composition may be for example, a food composition, a feed composition, or a feed additive composition.

The term used in the present description, "feed composition" refers to food fed to an animal. The feed composition refers to a substance supplying an organic or inorganic nutrient necessary for maintaining life of an animal or producing meat, milk, and the like. The feed composition may further comprise a nutritional component necessary for maintaining life of an animal or producing meat, milk, and the like. The feed composition may be produced as various types of feed known in the art, and specifically, may comprise concentrated feed, roughage and/or special feed.

The term used in the present description, "feed additive" includes substances added to feed on a purpose of various effects such as nutrient supplementation and weigh loss prevention, enhancement of digestibility of fiber in feed, improvement of oil quality, reproductive disorder prevention and fertility rate improvement, prevention of high temperature stress in summer, and the like. The feed additive of the present application correspond to supplementary feed under the Feed Management Act, and may further comprise mineral matter preparations such as sodium hydrocarbon, bentonite, magnesium oxide, complex mineral, and the like, mineral preparations which are trace mineral matters such as zinc, copper, cobalt, selenium and the like, vitamin preparations such as carotene, vitamin E, vitamins A, D and E, nicotinic acid, vitamin B complex, and the like, protective amino acid preparations such as methionine, lysine, and the like, protective fatty acid preparations such as fatty acid calcium salt, and the like, living cells and yeast agents such as probiotics (bacteria preparation), yeast culture, mold fermented product, and the like.

The term used in the present description, "food composition" includes all forms of functional food, nutritional supplements, health food and food additives, and the like, and the above types of food compositions may be prepared in various forms according to a common method known in the art.

There is no particular limitation on the type of food. Preferably, food may be in a form containing the *Schizochytrium* sp. CD03-7004 strain, and in addition, it may be used as an additive to other foods. Examples of food to which the above substances can be added include meat, sausages, bread, chocolates, candies, snacks, confectionery, pizza, ramen, other noodles, gum, dairy products including ice creams, various kinds of soups, beverages, tea, drinks, alcoholic beverages and vitamin complexes, and the like, and it includes all health foods in a conventional sense.

The composition of the present application may further comprise grains, for example, milled or crushed wheat, oats, barley, corn and rice; plant protein feed, for example, feed having soybeans and sunflower as a main component; animal protein feed, for example, blood meal, meat meal, bone meal and fish meal; sugars and dairy products, for example, dried components consisting of various kinds of powdered milk and whey powder, and in addition thereto, may further comprise a nutritional supplement, a digestion and absorption enhancer, a growth promoter, and the like.

The composition of the present application may be administered alone or administered in combination with other feed additive among an edible carrier. In addition, the composition may be directly mixed as top dressing or to feed or be easily administered as an oral formulation separate to feed into an animal. When the composition is administered separately to feed, it may be prepared as an intermediate release or sustained-release formulation, in combination of a pharmaceutically acceptable edible carrier as well known in the art. This edible carrier may be a solid or liquid, for example, corn starch, lactose, sucrose, soybean flake, peanut oil, olive oil, sesame oil and propylene glycol. When a solid carrier is used, the composition may be top dressing in a tablet, capsule, powder, troche or lozenge or undispersible form. When a liquid carrier is used, the composition may be a formulation of gelatin soft capsule, or syrup or suspension, emulsion or solution.

The composition of the present application may contain, for example, a preservative, stabilizer, wetting agent or emulsifier, cryoprotectant, or excipient, or the like. The cryoprotectant may be at least one selected from the group consisting of glycerol, trehalose, maltodextrin, nonfat dry milk and starch.

The preservative, stabilizer or excipient may be comprised in the composition in an effective dose sufficient for reducing deterioration of the *Schizochytrium* sp. strain comprised in the composition. In addition, the cryoprotectant may be comprised in the composition in an effective dose sufficient for reducing deterioration of the *Schizochytrium* sp. strain comprised in the composition when the composition is in a dried state.

The composition may be used by be adding to feed of an animal by sedimentation, spraying or mixing.

The composition of the present invention may be applied to various animal diets including mammals, birds, fish, crustaceans, cephalopods, reptiles and amphibians, but not limited thereto. For example, the mammal may include pigs, cows, sheep, goats, experimental rodents or pets, or the like, and the bird may include poultry, and the poultry may include chickens, turkey, ducks, geese, pheasants or quails, or the like, but not limited thereto. In addition, the fish may include commercial livestock fish and fry thereof, ornamental fish, and the like, and the crustacean may include shrimp, barnacles, and the like, but not limited thereto. Furthermore, the composition may be applied to the diet of zooplankton, a rotifer.

Other one aspect of the present application provides an antioxidant composition comprising the *Schizochytrium* sp. CD03-7004 strain, a culture of the strain, a dry matter of the culture, or a lysate of the dry matter.

The antioxidant composition may comprise the *Schizochytrium* sp. strain, a biomass derived from the strain, bio-oil derived from the strain, or a combination thereof.

Since the *Schizochytrium* sp. CD03-7004 strain of the present invention produces and/or comprises carotenoid-based antioxidant pigments with high antioxidant and anti-inflammatory efficacy, or has producing ability of carotenoid-based antioxidant pigments, it can be usefully used as an antioxidant composition.

The antioxidant composition may be a pharmaceutical composition, a food composition, a feed composition, or a feed additive composition.

The food composition may be a functional food composition.

In the functional food of the present invention, various auxiliary components may be further contained if necessary in addition to the active ingredients. The functional food of the present invention may comprise vitamins such as vitamin A, vitamin B1, vitamin B2, vitamin B3, vitamin B6, vitamin B12, folic acid, vitamin C, vitamin D3, vitamin E, and the like, and minerals such as copper, calcium, iron, magnesium, potassium, zinc and the like, or probiotics, or the like.

In addition, in the functional food of the present invention, various kinds of flavoring agents or natural carbohydrates or the like may be comprised as an additional component as same as common beverages. The flavoring agents may include natural sweeteners such as thaumatin and stevia extract, or synthetic sweeteners such as saccharin and aspartame, and the like. The natural carbohydrates may include monosaccharides such as glucose, fructose and the like, disaccharides such as maltose, sucrose, and the like, polysaccharides such as dextrin, cyclodextrin and the like, sugar alcohols such as xylitol, sorbitol, erythritol, and the like.

Other one aspect of the present application provides a method for preparing a biomass derived from a *Schizochytrium* sp. microalgae, comprising culturing *Schizochytrium* sp. CD03-7004 strain; and recovering a biomass from the strain, a culture of the strain, a dry matter of the culture, or a lysate of the dry matter.

The *Schizochytrium* sp. strain, culture of the strain, dry matter of the culture, and lysate of the dry matter are as described above.

The term used in the present description, "culturing" means to grow the microalga in an appropriately controlled environmental condition. The culturing process of the present application may be conducted according to a suitable medium and a culture condition known in the art. This culturing process may be easily adjusted and used by those skilled in the art according to the selected strain.

Specifically, the culturing of the *Schizochytrium* sp. strain of the present application may be performed under a heterotrophic condition, but not limited thereto.

The term used in the present description, "heterotrophic" is a nutritional method depending on an organic matter obtained from an energy source or nutrient source outside the body, and is a term corresponding to autotrophic, and it may be interchangeably used with a term 'dark culture'.

The culturing a *Schizochytrium* sp. strain is not particularly limited thereto, but may be performed by known batch culturing method, continuous culture method, fed-batch culturing method, and the like. Any medium and other culturing condition used in the culturing of the strain of the present application may be used without particular limitation as long as it is a medium used for culturing a common strain. Specifically, the strain of the present application may be cultured by adjusting temperature, pH and the like, under an aerobic condition in a common medium containing a suitable carbon source, a nitrogen source, a phosphorus source, an inorganic compound, an amino acid and/or a vitamin, and the like.

Specifically, an appropriate pH (for example, pH 5 to 9, specifically, pH 6 to 8, most specifically, pH 6.8) may be adjusted using a basic compound (e.g.: sodium hydroxide, potassium hydroxide or ammonia) or acidic compound (e.g.: phosphate or sulfate), but not limited thereto.

In addition, in order to maintain the aerobic condition of the culture, oxygen or oxygen-containing gas may be injected into the culture, or in order to maintain anaerobic and microaerobic conditions, nitrogen, hydrogen or carbon dioxide may be injected without injection of gas, but not limited thereto.

Furthermore, the culturing temperature may be maintained at 20 to 45°C or 25 to 40°C, and may be cultured for about 10 to 160 hours, but not limited thereto. In addition, during the culturing, an antifoaming agent such as fatty acid polyglycol ester may be used to inhibit bubble formation, but not limited thereto.

The carbon source comprised in the medium used in the culturing the *Schizochytrium* sp. strain may be any one or more selected from the group consisting of glucose, fructose, maltose, galactose, mannose, sucrose, arabinose, xylose and glycerol, but any carbon source used for culturing microalgae is not limited thereto.

The nitrogen source comprised in the medium used in the culturing the *Schizochytrium* sp. strain may be i) any one or more of organic nitrogen sources selected from the group consisting of yeast extract, beef extract, peptone and tryptone, or ii) any one or more of inorganic nitrogen sources selected from the group consisting of ammonium acetate, ammonium nitrate, ammonium chloride, ammonium sulfate, sodium nitrate, urea and MSG (Monosodium glutamate), but any nitrogen source used for culturing a microalgae is not limited thereto.

The medium used in the culturing the *Schizochytrium* sp. strain, may separately comprise or mix and comprise potassium dihydrogen phosphate, dipotassium hydrogen phosphate, as a phosphorus source, and a sodium-containing salt corresponding thereto, and the like, but not limited thereto.

The recovering biomass from the strain, culture of the strain, dry matter of the culture, or lysate of the dry matter may collect targeted biomass using an appropriate method known in the art. For example, centrifugation, filtration, anion exchange chromatography, crystallization and HPLC and the like may be used, and it may further comprise a purification process.

Other aspect of the present invention provides a method for producing bio-oil derived from a *Schizochytrium* sp. strain, comprising culturing *Schizochytrium* sp. CD03-7004 strain; and recovering lipids from the strain, a culture of the strain, a dry matter of the culture, or a lysate of the dry matter.

The *Schizochytrium* sp. strain, bio-oil, culture of the strain, dry matter of the culture, and lysate of the dry matter, and culturing the strain are as described above.

The recovering lipid from the strain, culture of the strain, dry matter of the culture, or lysate of the dry matter may collect targeted lipids using an appropriate method known in the art. For example, centrifugation, filtration, anion exchange chromatography, crystallization and HPLC and the like may be used, and it may further comprise a purification process.

For example, lipids and lipid derivatives such as fat aldehyde, fat alcohol and hydrocarbon (for example, alkane) may be extracted with a hydrophobic solvent such as hexane. The lipids and lipid derivatives may be also extracted using a method for liquefaction, oil liquefaction and supercritical $CO_2$ extraction, and the like. In addition, a known microalgal lipid recovery method includes for example, a method i) for harvesting cells by centrifugation and washing them with distilled water, and then drying them by freeze-drying, and ii) for pulverizing the obtained cell powder, and then extracting lipids with n-hexane (Miao, X and Wu, Q, Biosource Technology (2006) 97:841-846).

Other aspect of the present application
provides a method for producing antioxidant pigments comprising culturing *Schizochytrium* sp. CD03-7004 strain; and extracting carotenoid-based antioxidant pigments from a culture of the strain.

The antioxidant pigments may be beta-carotene or canthaxanthin, but not limited thereto.

The culturing the *Schizochytrium* sp. strain is as described above.

The antioxidant pigments, beta-carotene and canthaxanthin are as described above.

The antioxidant pigments may be extracted or separated and purified by a common method in the art. The antioxidant pigments present in a microbial cell may be extracted using an organic solvent, for example, hexane, acetone, chloroform, ethyl acetate, methyl alcohol, dichloromethane, cyclohexane, ethanol, benzene, carbon disulfide, diethyl ether or a mixed solvent thereof.

[ADVANTAGEOUS EFFECTS]

The present invention relates to a novel *Schizochytrium* sp. strain producing a biomass comprising a high protein content, antioxidant pigments and omega-3 fatty acid, and a method for producing a biomass containing high protein content, antioxidant pigments and omega-3 fatty acid using thereof, and since the novel *Schizochytrium* sp. strain of the present invention has a characteristic of simultaneously having a high protein content comprising amino acids such as glutamic acid, arginine and aspartic acid, and the like, antioxidant pigments such as beta-carotene and, and omega-3 unsaturated fatty acid components including eicosapentaenoic acid and docosahexaenoic acid in cells, the strain itself or a biomass and bio-oil produced by culture and fermentation of the strain can be usefully used as a feed composition or food composition, or the like.

[BRIEF DESCRIPTION OF THE DRAWINGS]

FIG. 1 is a picture showing a difference in color, as a result of pure isolation culture of the *Schizochytrium* sp. CD03-7004 mutant strain and wild-type *Schizochytrium* sp. strain CD01-5000.

FIG. 2 is a picture showing the size of the amplified DNA fragments of the wild-type CD01-5000 strain and the mutant CD03-7004 strain after PCR reaction was performed using a primer set that amplifies a DNA fragment comprising an additional 15 bp of nucleotide sequence in the mutant CD03-7004 strain compared to the wild-type CD01-5000 strain.

[DETAILED DESCRIPTION OF THE INVENTION]

Hereinafter, the present invention will be described in more detail by the following examples. However, these examples are intended to illustrate the present invention only, but the scope of the present invention is not limited by these examples.

**Example 1. Development of a novel *Schizochytrium* sp. CD03-7004 mutant strain through a gamma ray irradiation method**

Purely separated wild-type *Schizochytrium* sp. CD01-5000 strain (Accession number KCTC 14344BP) was cultured in a modified-GYEP (glucose 10 g/L, yeast extract 1 g/L, peptone 1 g/L, $MgSO_4 \cdot 7H_2O$ 2 g/L, $H_3BO_3$ 5.0 mg/L, $MnCl_2$ 3.0 mg/L, $CuSO_4$ 0.2 mg/L, $NaMo_4 \cdot 2H_2O$ 0.05 mg/L, $CoSO_4$ 0.05 mg/L, $ZnSO_4 \cdot 7H_2O$ 0.7 mg/L) medium comprising glucose 30 g/L for about 24 hours or more to reach an early exponential phase, and then microbial cells were harvested by centrifuging the culture solution sample. The harvested microbial cells were utilized for gamma ray irradiation by suspending them in 0.1M Phosphate Buffer Solution comprising NaCl 1.0% so that the number of cells was to be about $10^9$ cells/mL. A gamma ray irradiation experiment was conducted in Korea Atomic Energy Research Institute, Advanced Radiation Technology Institute, and gamma rays of 2000~5000 GY doses were irradiated. The microalgal culture solution sample to which gamma rays were irradiated was under a recovery process for 0/N in a dark room, and then was smeared in a GYEPP medium comprising agar 20 g/L and cultured at 30°C for about 5 days, and then the number of growing colonies was counted, and the death rate by gamma ray dose was measured.

Death rate (%) = [{(number of colonies of untreated group) - (number of colonies of treated group)}/(number of colonies of untreated group)] $\times$ 100 [Calculation Formula 1]

[Table 1]

| Gamma ray dose (kGY) | Number of growing colonies (EA) | Death rate (%) |
| --- | --- | --- |
| 2.0 | $\geq$ 250 | - |
| 3.0 | $\geq$ 23 | 90.8 |
| 4.0 | $\geq$ 7 | 98.2 |
| 4.5 | 0 | 100 |
| 5.0 | 0 | 100 |
| Radiation untreated group | $\geq$ 250 | 0 |

As a result, as shown in Table 1, the number of growing colonies depending on the gamma ray irradiation dose, and an appropriate dose at which the CFU value of the number of living cells was reduced by 95% or more were confirmed. Specifically, a result that 0%, 90.8%, 98.2%, 100%, 100% were killed at a gamma ray irradiation dose of 2.0, 3.0, 4.0, 4.5, 5.0 kGY, respectively, was shown. Under a condition of gamma ray dose irradiation of 4.5 GY or more, all were killed, so microalgal colonies could not be secured, and the gamma ray dose condition of 4.0 kGY showing the death rate of 98.2% was selected.

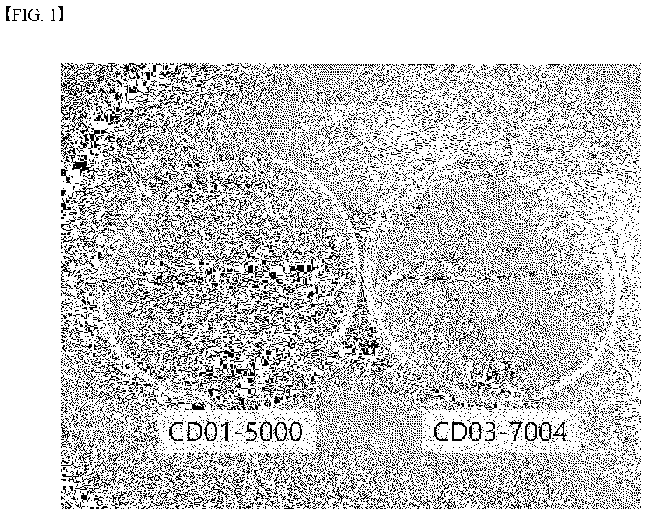

After irradiating gamma rays of 4.0 kGY dose into the novel microalgal strain CD01-5000 by the same method, it was cultured in a GYEP medium. During culturing, colonies which could be cultured were selected, and subculturing was performed in the same medium and culture environment conditions. Morphologically reddish colonies between passages were selected and pure separated and cultured with a single cell line, and then the result of photographing them was shown in FIG. 1. The *Schizochytrium* sp. strain was named *Schizochytrium* sp. CD03-7004 strain, and deposited to Korean Collection for Type Cultures (KCTC) which is an international depository authority under the Budapest Treaty on June 20, 2022 and was given an accession number KCTC15006BP.

**Example 2. Analysis of crude fat and fatty acid contents in the *Schizochytrium* sp. CD03-7004 mutant strain culture solution**

**Example 2-1. Culturing of CD01-5000 strain and CD03-7004 strain**

In order to compare components of the wild-type *Schizochytrium* sp. CD01-5000 strain and the mutant *Schizochytrium* sp. CD03-7004 strain culture solution obtained through gamma ray irradiation, dry microbial cells obtained by drying a culture solution in which each strain was fermented and cultured were used as a component analysis sample. For pre-culturing before the present culturing of 30L scale, each strain was inoculated in a GYEP medium comprising working volume 50 ml and glucose 30 g/L in a 500 ml flask, and cultured in a shaking incubator at 30°C, 180 rpm for about 20 hours. The pre-culture was inoculated in a 30L fermentor comprising a medium under the same condition, and fermented and cultured at a total working volume of 20L. Glucose corresponding to 20% of the working volume was continuously added under the condition of 30°C, 500 rpm, 0.5-1 vvm, pH 5-7, and used for cell culture, and it was added so that the glucose concentration at this time was maintained at a level of 20 g/L. When the supplied carbon source, glucose was exhausted, the culture was terminated. The culture solution in which culturing was completed was dried so that the moisture content was to be about 5~8% using a freeze dryer, and the obtained dry microbial cells were used for analysis of crude fat, crude protein and carotenoid components.

**Example 2-2. Analysis of crude fat and fatty acid contents of CD01-5000 strain and CD03-7004 strain culture solution**

In order to analyze crude fat and fatty acid contents of the culture solution of the wild-type *Schizochytrium* sp. CD01-5000 strain and the mutant *Schizochytrium* sp. CD03-7004 strain of the present invention, the following method was used.

Specifically, in order to hydrolyze cell walls of the microalgal microbial cells, a 8.3M hydrochloric acid solution was added to the dried microalgal microbial cells 2g obtained in Example 1-1 was added and heated at 80°C, and then ethyl ether 30 mL and petroleum ether 20 mL were added and mixed for 30 seconds, and then a process of centrifugation was repeated three times or more. The separated solvent layer was recovered, and moved into a round-flask of which weight was measured in advance, and nitrogen was injected to remove the solvent, and then was cooled and maintained in a constant amount. The weight of the dried oil was measured by a value in which the weight of the empty flask was subtracted from the weight of the flask after drying, and the total oil content was calculated. The DHA content comprised in the oil was shown by pretreating methanolic 0.5N NaOH and 14% boron trifluoride-methanol (BF3) and measuring with gas chromatography.

[Calculation formula 2]

$$\text{Total oil content (\%)} = (\text{*oil g/dried microbial cell amount g}) \times 100$$

*oil g: flask weight after acid hydrolysis and solvent removal - empty flask weight

The "biomass" of Table 1 below means a concentration of the microbial cell in the culture solution, and it may be interchangeably used with DCW (dry cell weight).

[Table 2]

| | *Schizochytrium* sp. CD01-5000 | *Schizochytrium* sp. CD03-7004 |
|---|---|---|
| **Culturing time (hr)** | 26.6 | 28.3 |
| **O.D (680nm)** | 166.9 | 124.6 |
| **DCW (g/L)** | 107.9 | 105 |
| **Crude fat content in biomass (%)** | 25.3 | 14.4 |

(continued)

| Fatty acids in crude fats (%) | | |
|---|---|---|
| - C20:5 n-3 | 0.6 | 2.1 |
| - C22:6 n-3 | 41.3 | 49.4 |
| (In the above table, C20:5 n-3 means eicosapentaenoic acid (EPA), and C22:6 n-3 means docosahexaenoic acid (DHA).) | | |

As a result, as shown in Table 2, as the experimental result, both the wild-type strain CD01-5000 strain and the mutant CD03-7004 strain digested all of the supplied carbon source, glucose, within about 30 hours, and produced biomass at a level of about 100 g/L, thereby showing similar biomass growth. In case of the CD03-7004 strain, the crude fat content in the biomass was 14.4% and lower compared to the CD01-5000 strain, but the eicosapentaenoic acid (EPA, C20:5 n-3) content was shown as 2.1% and the docosahexaenoic acid (DHA, C22:6 n-3) content was shown as 49.4%, so it was confirmed that they were significantly higher compared to the CD01-5000 strain. In other words, it was confirmed that the CD03-7004 strain contained a high content of omega-3 fatty acids of 50% or more in the crude fat.

**Example 3. Analysis of crude protein and amino acid contents of *Schizochytrium* sp. CD03-7004 mutant strain culture solution**

In order to analyze crude fat and amino acid contents of the culture solution dry microbial cells of the wild-type *Schizochytrium* sp. CD01-5000 strain and the mutant *Schizochytrium* sp. CD03-7004 strain of the present invention, the following method was used.

Specifically, 1g of the dried microalgal microbial cells obtained in Example 1-1 was placed in a tube for analysis using a Kjeldahl device (Kjeldahl system, Kjeltec 2100), and 95% sulfuric acid 12~15 mL and a catalyst were added and degraded in a decomposer, and then the degradation tube was equipped to an autosampler. The crude protein content of microalgal dry biomass was measured by the method of titrating this with a 0.1N hydrochloric acid solution and calculating the nitrogen content automatically, after cooling and collecting gaseous ammonia generated by heating and distilling with caustic soda and steam in the Kjeldahl device. After acid hydrolysis of 0.5 to 1 g of the dried microalgal microbial cells obtained in Example 1-1 with a 6N HCl solution, total amino acid analysis was performed using liquid chromatography. The analysis result of individual amino acids was standardized by the amount of used microalgal dry microbial cells to calculate the content ratio of individual amino acids in the dry microbial cells, and by adding the content ratios of all the amino acids detected as a result of the analysis, the total amino acid content ratio in the microbial cells was calculated.

[Table 3]

|  | *Schizochytrium* sp. CD01-5000 | *Schizochytrium* sp. CD03-7004 |
|---|---|---|
| **Crude protein amount in biomass (%)** | 59.7 | 75.1 |
| **Amino acid amount in biomass (%)** | | |
| Aspartic acid | 3.98 | 3.86 |
| Threonine | 1.82 | 1.75 |
| Serine | 2.23 | 1.99 |
| Glutamic acid | 13.57 | 21.30 |
| Glycine | 1.87 | 1.86 |
| Alanine | 2.50 | 2.53 |
| Cysteine | 0.59 | 0.52 |
| Valine | 1.90 | 2.21 |
| Methionine | 0.76 | 0.70 |
| Isoleucine | 1.16 | 1.34 |
| Leucine | 2.80 | 2.72 |
| Tyrosine | 1.24 | 1.10 |
| Phenylalanin e | 1.52 | 1.66 |

(continued)

|  | *Schizochytrium* sp. CD01-5000 | *Schizochytrium* sp. CD03-7004 |
|---|---|---|
| Lysine | 2.50 | 2.61 |
| Histidine | 0.72 | 0.84 |
| Arginine | 5.59 | 8.27 |
| Proline | 0.77 | 0.16 |
| (In the above table, the amount of amino acids in biomass means the amount (g) of each amino acid in biomass (kg) expressed as a ratio (%).) | | |

As a result, as shown in Table 3, it was confirmed that the crude protein content in the CD03-7004 mutant strain culture solution dry microbial cells was 75.1%, and was higher than the crude protein content in the wild-type CD01-5000 strain culture solution dry microbial cells of 59.7%. In addition, it was confirmed that in the amino acid content in the CD03-7004 mutant strain culture solution dry microbial cells, glutamic acid was the highest, and it was high in the order of phenylalanine, arginine, aspartic acid, and lysine.

**Example 4. Analysis of antioxidant pigment content of Schizochytrium sp. CD03-7004 mutant strain culture solution**

In order to analyze the antioxidant pigment content of the wild-type *Schizochytrium* sp. CD01-5000 strain and the mutant *Schizochytrium* sp. CD03-7004 strain culture solution of the present invention, the following method was used.

Specifically, by weighing an appropriate amount of the dried microalgal microbial cells obtained in Example 1-1, they were extracted and concentrated with an extraction solution consisting of hexane, acetone, chloroform and ethyl acetate, and analyzed with high performance liquid chromatography (HPLC), and the result was shown in Table 4 below.

[Table 4]

| Antioxidant pigments | *Schizochytrium* sp. CD01-5000 | *Schizochytrium* sp. CD03-7004 |
|---|---|---|
| Beta-carotene (mg/kg) | Non-detected | 2.77 |
| Canthaxanthin (mg/kg) | 6.22 | 112.09 |

As a result, as shown in Table 4, in the culture solution dry microbial cells in which the *Schizochytrium* sp. CD01-5000 strain was cultured, beta-carotene was undetected, and the canthaxanthin content was 6.22 mg/kg, whereas in the culture solution dry microbial cells in which the *Schizochytrium* sp. CD03-7004 strain was cultured, the beta-carotene content was shown as 2.77mg/kg, and the canthaxanthin content was shown as 112.09 mg/kg, and therefore, it was confirmed that the *Schizochytrium* sp. CD03-7004 mutant strain of the present invention produced high levels of antioxidant pigments of beta-carotene and canthaxanthin.

**Example 5. Derivation of markers for distinguishing CD03-7004 mutant strain from wild type CD01-5000 strain**

By comparing the whole genome sequences of the wild-type *Schizochytrium* sp. CD01-5000 strain and the *Schizochytrium* sp. CD03-7004 mutant strain, specific sequence was identified that were only present in strain CD03-7004 mutant strain. Then, PCR markers were prepared based on the corresponding the nucleotide sequence.

Specifically, the genome of the CD03-7004 mutant strain was confirmed to have an additional 15 base pairs ("bolded portion" of the sequence in SEQ ID NO: 1 below) when compared to the genome of the wild-type CD01-5000 strain.

[DNA fragment sequence to be amplified from CD03-7004 mutant strain (SEQ ID NO: 1)]

TTTCAGACTGCTTTTTGCTTTTTGCTTGCTTGCTTTT**TGCTTGCTTGCTTTT**G

GCTTGCTTTCTTTTTGCTTCTTCCTGCTTGATCCGGTGAAGAAGAACGGAGCGAACTA

AAAGAAAAGAGTCAATCCGAAGAGAG

[DNA fragment sequence to be amplified from wild-type CD01-5000 strain (SEQ ID NO: 2)]

TTTCAGACTGCTTTTTGCTTTTTGCTTGCTTGCTTTTGGCTTGCTTTCTTTTTGC

TTCTTCCTGCTTGATCCGGTGAAGAAGAACGGAGCGAACTAAAAGAAAAGAGTCAAT

CCGAAGAGAG

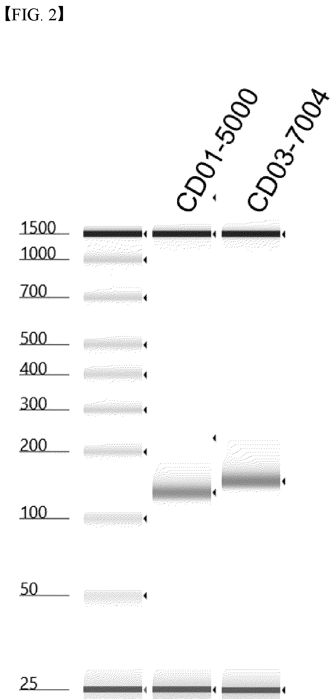

Primer A: 5'-TTTCAGACTGCTTTTTGCTTTTTG-3' (SEQ ID NO: 3) and primer B: 5'-CTCTCTTCGGATTGA CTCTTTTTCT-3' (SEQ ID NO: 4) were selected to amplify these DNA fragment sequences, and PCR amplification reaction was carried out using them. The PCR reaction was performed using a reaction solution containing taq polymerase and denatured at 95°C for 5 minutes. Then, 95°C for 10 s denaturation, 50°C for 10 s annealing, and 72°C for 15 s polymerization were repeated 35 times, and then polymerization was performed at 72°C for 5 min. The amplified reactant from the PCR process were electrophoresed on 1.7% agarose gel to determine the size of the amplified DNA fragment, and the result is shown in FIG. 2.

As a result, as shown in FIG. 2, the DNA fragment amplified in the CD03-7004 mutant strain was about 140 bp in size, and it was confirmed that it was different from the wild-type CD01-5000 strain in which the DNA fragment of about 120 bp was amplified.

Thus, based on the above results, it can be seen that primer A: 5'-TTTCAGACTGCTTTTTGCTTTTTG-3' (SEQ ID NO: 3) and primer B: 5'-CTCTCTTCGGATTGACTCTTTTTCT-3' (SEQ ID NO: 4) can be used to screen (identify) the CD03-7004 mutant strain.

From the above description, those skilled in the art to which the present application pertains will be able to understand that the present application may be implemented in other specific forms without changing the technical spirit or essential characteristics thereof. In this regard, it should be understood that the examples described above are illustrative and not restrictive in all respects. The scope of the present application should be construed as including all changes or modified forms derived from the meaning and scope of the claims to be described later and equivalent concepts thereof rather than the detailed description.

[Accession number]

Name of Depository Authority: Korea Research Institute of Bioscience and Biotechnology Korea Collection for Type Culture (KCTC)
Accession number: KCTC15006BP
Deposit date: 20220620

# EP 4 556 553 A1

INTERNATIONAL FORM

# RECEIPT IN THE CASE OF AN ORIGINAL DEPOSIT

issued pursuant to Rule 7.1

TO: **CJ CheilJedang Corporation**

CJ CheilJedang Corporation

330, Dongho-ro, Jung-gu, Seoul

Republic of Korea

| I.  IDENTIFICATION OF THE MICROORGANISM | |
|---|---|
| Identification reference given by the DEPOSITOR: <br><br> *Schizochytrium* sp. **CD03-7004** | Accession number given by the INTERNATIONAL DEPOSITARY AUTHORITY: <br><br> **KCTC 15006BP** |

| II.  SCIENTIFIC DESCRIPTION AND/OR PROPOSED TAXONOMIC DESIGNATION |
|---|
| The microorganism identified under I above was accompanied by: <br><br> [  ] a scientific description <br><br> [  ] a proposed taxonomic designation <br><br> (Mark with a cross where applicable) |

| III.  RECEIPT AND ACCEPTANCE |
|---|
| This International Depositary Authority accepts the microorganism identified under I above, which was received by it on **June 20, 2022.** |

| IV.  RECEIPT OF REQUEST FOR CONVERSION |
|---|
| The microorganism identified under I above was received by this International Depositary Authority on and a request to convert the original deposit to a deposit under the Budapest Treaty was received by it on |

| V.  INTERNATIONAL DEPOSITARY AUTHORITY | |
|---|---|
| Name: **Korean Collection for Type Cultures** <br><br> Address: Korea Research Institute of Bioscience and Biotechnology (KRIBB) 181, Ipsin-gil, Jeongeup-si, Jeollabuk-do 56212 Republic of Korea | Signature(s) of person(s) having the power to represent the International Depositary Authority or of authorized official(s): <br><br> *Song-Gun Kim* <br><br> KIM, Song-Gun, Director Date: **June 20, 2022** |

Form BP/4 (KCTC Form 17)                                                                    sole page

**Claims**

1. Novel *Schizochytrium* sp. CD03-7004 strain (Accession number KCTC15006BP).

2. The strain according to claim 1, wherein the strain has producing ability of carotenoid-based antioxidant pigments.

3. The strain according to claim 2, wherein the antioxidant pigments are beta-carotene or canthaxanthin.

4. The strain according to any one claim of claim 1 to claim 3, wherein the strain has producing ability of omega-3 unsaturated fatty acids.

5. The strain according to claim 4, wherein the omega-3 unsaturated fatty acids are docosahexaenoic acid (DHA) and eicosapentaenoic acid (EPA).

6. The strain according to claim 1, wherein the genomic DNA of the strain comprises the nucleotide sequence of SEQ ID NO: 1.

7. The strain according to claim 1, wherein the strain can be identified using a primer set comprising a primer having the nucleotide sequence of SEQ ID NO: 3 and a primer having the nucleotide sequence of SEQ ID NO: 4.

8. A biomass comprising the strain of claim 1, a culture solution of the strain, a dry matter of the culture solution, or a lysate of the dry matter.

9. The biomass according to claim 8, wherein the biomass comprises carotenoid-based antioxidant pigments.

10. The biomass according to claim 9, wherein the antioxidant pigments are beta-carotene or canthaxanthin.

11. The biomass according to any one claim of claim 8 to claim 10, wherein the biomass contains omega-3 unsaturated fatty acid of 42% or more based on the total weight of fatty acid.

12. The biomass according to claim 11, wherein the omega-3 unsaturated fatty acids are docosahexaenoic acid (DHA) and eicosapentaenoic acid (EPA).

13. The biomass according to any one claim of claim 8 to claim 10, wherein the biomass comprises proteins of 60% or more based on the dry weight.

14. A feed composition comprising the strain of claim 1, a culture solution of the strain, a dry matter of the culture solution, or a lysate of the dry matter.

15. A food composition comprising the strain of claim 1, a culture solution of the strain, a dry matter of the culture solution, or a lysate of the dry matter.

16. An antioxidant composition comprising the strain of claim 1, a culture solution of the strain, a dry matter of the culture solution, or a lysate of the dry matter as an active ingredient.

17. A method for producing antioxidant pigments, comprising

    culturing the strain of claim 1; and
    extracting carotenoid-based antioxidant pigments from a culture of the strain.

18. The method for producing antioxidant pigments according to claim 17, wherein the antioxidant pigments are beta-carotene or canthaxanthin.

【FIG. 1】

【FIG. 2】

## INTERNATIONAL SEARCH REPORT

| International application No. |
|---|
| **PCT/KR2023/012051** |

### A. CLASSIFICATION OF SUBJECT MATTER

**C12N 1/12**(2006.01)i; **C12P 23/00**(2006.01)i; **C12P 7/6434**(2022.01)i; **C12P 7/6432**(2022.01)i; **C12P 21/00**(2006.01)i; **A23K 10/16**(2016.01)i; **A23L 29/00**(2016.01)i; C12R 1/89(2006.01)n

According to International Patent Classification (IPC) or to both national classification and IPC

### B. FIELDS SEARCHED

Minimum documentation searched (classification system followed by classification symbols)

C12N 1/12(2006.01); C12N 1/00(2006.01); C12N 1/14(2006.01); C12P 23/00(2006.01); C12P 7/64(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 스키조키트리움 속(Schizochytrium sp.), CD03-7004, 오메가-3(omega-3), DHA, EPA, 베타카로틴(beta-carotene), 칸타잔틴(canthaxanthin), 단백질(protein), 바이오매스(biomass)

### C. DOCUMENTS CONSIDERED TO BE RELEVANT

| Category* | Citation of document, with indication, where appropriate, of the relevant passages | Relevant to claim No. |
|---|---|---|
| X | KR 10-2022-0080594 A (CJ CHEILJEDANG CORPORATION) 14 June 2022 (2022-06-14)<br>See paragraphs [0025], [0029], [0031]-[0032], [0035] and [0040]; and examples 3-4 and 6. | 1-5,8-18 |
| A | | 6-7 |
| A | KR 10-1847551 B1 (INDUSTRIAL COOPERATION FOUNDATION JEONBUK NATIONAL UNIVERSITY et al.) 10 April 2018 (2018-04-10)<br>See entire document. | 1-18 |
| A | PARK, Hansung et al. Enhanced production of carotenoids using a Thraustochytrid microalgal strain containing high levels of docosahexaenoic acid-rich oil. Bioprocess and Biosystems Engineering. 2018, vol. 41, no. 9, pp. 1355-1370 (inner pp. 1-16).<br>See entire document. | 1-18 |
| A | US 2021-0340583 A1 (QU, Hanpeng) 04 November 2021 (2021-11-04)<br>See entire document. | 1-18 |

☑ Further documents are listed in the continuation of Box C.          ☑ See patent family annex.

| | |
|---|---|
| \* Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **08 November 2023** | **09 November 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

International application No.

**PCT/KR2023/012051**

### C. DOCUMENTS CONSIDERED TO BE RELEVANT

| Category* | Citation of document, with indication, where appropriate, of the relevant passages | Relevant to claim No. |
|---|---|---|
| A | KR 10-2008-0111586 A (LEE, Joung Yeoul) 24 December 2008 (2008-12-24)<br>See entire document. | 1-18 |

Form PCT/ISA/210 (second sheet) (July 2022)

## INTERNATIONAL SEARCH REPORT

International application No.

**PCT/KR2023/012051**

| Box No. I | Nucleotide and/or amino acid sequence(s) (Continuation of item 1.c of the first sheet) |
|---|---|

1.  With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

        ☐    accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

**INTERNATIONAL SEARCH REPORT**
Information on patent family members

International application No.

**PCT/KR2023/012051**

| Patent document cited in search report | | | Publication date (day/month/year) | Patent family member(s) | | | Publication date (day/month/year) |
|---|---|---|---|---|---|---|---|
| KR | 10-2022-0080594 | A | 14 June 2022 | KR | 10-2550213 | B1 | 30 June 2023 |
| | | | | WO | 2022-124590 | A1 | 16 June 2022 |
| KR | 10-1847551 | B1 | 10 April 2018 | None | | | |
| US | 2021-0340583 | A1 | 04 November 2021 | CN | 110846346 | A | 28 February 2020 |
| | | | | EP | 3896167 | A1 | 20 October 2021 |
| | | | | KR | 10-2022-0088912 | A | 28 June 2022 |
| | | | | WO | 2021-104165 | A1 | 03 June 2021 |
| KR | 10-2008-0111586 | A | 24 December 2008 | None | | | |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020220103398

- US 5130242 A

**Non-patent literature cited in the description**

- **PALOZZA, P et al.** *Arch. Biochem. Biophys.*, 1992, vol. 297, 291-295
- **PALOZZA, P et al.** *Arch. Biochem. Biophys.*, 1996, vol. 325, 145-151
- **TANAKA, T. et al.** *Cancer Res.*, 1995, vol. 55, 4059-4064

- **TANAKA, T. et al.** *Carcinogenesis*, 1995, vol. 16, 2957-2963
- **KATSUMURA, N. et al.** *Nutr. Cancer*, 1996, vol. 26, 203-208
- **MIAO, X ; WU, Q.** *Biosource Technology*, 2006, vol. 97, 841-846